# FASCICULE DE BREVET EUROPEEN

(11) **EP 0 526 289 B1**
(45) Date de publication et mention de la délivrance du brevet: **16.10.1996**
(21) Numéro de dépôt: 92401962.3
(22) Date de dépôt: 08.07.1992
(51) Int. Cl.: A61K 7/00

(54) **Procédé de fabrication d'une composition cosmétique pour application sur les cheveux, composition obtenue par ce procédé et procédé de traitement cosmétique à l'aide de ladite composition**
Verfahren zur Herstellung von kosmetische Haarpflegemitteln sowie die Zusammensetzung und Verfahren zur kosmetischer Anwendung dieser Zusammensetzung
Method for the manufacture of a cosmetic composition for hair treatment, and its obtained composition and a method of cosmetic treating using this composition

(30) Priorité: 24.07.1991 FR 9109340
(43) Date de publication de la demande: 03.02.1993
(73) Titulaire: L'OREAL, F-75008 Paris (FR)
(72) Inventeur: Hansenne-Richoux, Isabelle, F-75017 Paris (FR)
(74) Mandataire: Peuscet, Jacques

(56) Documents cités:
- EP-A- 0 043 327
- EP-A- 0 155 806
- FR-A- 2 315 991
- FR-A- 2 597 345
- FR-A- 2 597 367

## Description

La présente invention concerne un procédé de fabrication d'une composition cosmétique pour application sur les cheveux, la composition cosmétique obtenue par ce procédé et un procédé de traitement cosmétique à l'aide de ladite composition.

Il est bien connu que les cheveux sont sensibilisés ou fragilisés à des degrés divers sous l'action des agents atmosphériques et/ou de certains traitement cosmétiques, tels que permanentes, teintures ou décolorations. Les cheveux sensibilisés ou fragilisés deviennent difficiles à démêler et à coiffer aussi bien à l'état mouillé qu'à l'état sec. De plus, ils sont rêches au toucher, n'ont plus un aspect lisse ou brillant et se chargent d'électricité statique.

On a donc cherché à appliquer sur la chevelure des compositions, qui permettent de remédier à ces différents problèmes.

Il est connu, de très longue date, d'utiliser des huiles et des corps gras pour redonner aux cheveux de la douceur et du brillant ; l'application de ces composés est généralement suivie d'un shampooing pour éliminer des cheveux l'excédent d'huile ou de corps gras. Cependant, l'utilisation des huiles et corps gras amollit et alourdit les cheveux et il est impossible d'obtenir par la suite une coiffure ayant de la tenue et du volume.

On a déjà proposé d'utiliser des compositions contenant des huiles de silicone. Celles-ci permettent d'obtenir des cheveux brillants, mais leur usage prolongé ou en grande quantité a l'inconvénient de donner aux cheveux un aspect gras.

On utilise couramment des compositions aqueuses contenant des agents tensioactifs cationiques, que l'on applique sur la chevelure et qu'on laisse agir pendant quelques minutes avant de rincer les cheveux. Les agents tensioactifs cationiques améliorent le démelage et le coiffage, mais présentent des inconvénients : ils ont tendance à alourdir la chevelure et à lui donner un aspect gras. De plus, la chevelure a tendance à se resalir rapidement. Ces inconvénients sont d'autant plus accentués que les cheveux traités sont plus fins et plus sensibilisés.

Il est également connu d'utiliser des compositions contenant à la fois des agents tensioactifs cationiques et des huiles ou corps gras. Ces compositions peuvent avoir de bonnes propriétés cosmétiques et rhéologiques, mais elles ont tendance à former un dépôt sur les cheveux.

Il est, par ailleurs, connu que certains lipides amphiphiles sont susceptibles de former, par agitation en présence d'une phase aqueuse, une phase lamellaire lipidique hydratée conduisant à des vésicules ; le brevet français n° 2 315 991 décrit, en particulier, le cas où le lipide amphiphile est un lipide ionique. Ces vésicules sont constituées d'un ou plusieurs feuillets concentriques (couches bimoléculaires ou multimoléculaires de lipides encapsulant une phase aqueuse interne). Le(les) lipides(s) ionique(s) utilisé(s) est (sont), de façon connue, des lipides amphiphiles d'origine naturelle ou synthétique comportant, par molécule, une (ou plusieurs) longue(s) chaîne(s) hydrocarbonée(s). Les nombreux procédés de fabrication des vésicules de lipides ioniques sont bien connus. Dans un premier type de procédé (voir notamment US-A-4 772 471), on dissout les lipides amphiphiles dans un solvant, puis on évapore le solvant ; on introduit ensuite sous agitation là phase aqueuse à encapsuler et on soumet le tout à une agitation énergique. Dans un deuxième type de procédé (voir notamment FR-A-2 315 991), on évite l'emploi d'un solvant : dans une première étape, on met en contact le(s) lipide(s) amphiphile(s) ionique(s) fondu(s) (70-95°C par exemple) avec la phase aqueuse à encapsuler sous forte agitation jusqu'à formation des vésicules ; dans une seconde étape, on ajoute une phase aqueuse de dispersion, identique ou différente de la phase aqueuse à encapsuler en poursuivant l'agitation.

Dans un troisième type de procédé ( voir notamment FR 89 13358), on dissout le(s) lipide(s) amphiphile(s) ionique(s) dans un solvant organique non miscible à l'eau ; on ajoute la phase organique ainsi obtenue a une phase aqueuse en quantités telles que l'on obtienne par forte agitation une dispersion du type huile - dans - eau ; on évapore le solvant sous forte agitation, la phase continue de la dispersion restant en permanence la phase aqueuse, la dispersion pouvant être ensuite éventuellement concentrée.

Selon la présente demande, on a trouvé que l'on améliore les caractéristiques de traitement des cheveux à l'aide d'une composition aqueuse contenant des silicones et des lipides, lorsqu'on utilise comme lipides des lipides amphiphiles ioniques susceptibles de former des vésicules et que l'on prépare la composition à l'aide d'un procédé connu de fabrication de vésicules ioniques, dans lequel on mélange le lipide amphiphile ionique et la silicone avant le processus de formation des vésicules.

On a vérifié, notamment par microscopie électronique, que la composition ainsi préparée contient des vésicules stables.

On a montré par des essais comparatifs que le fait d'introduire la silicone dans le lipide amphiphile ionique avant le processus de formation des vésicules permet d'obtenir de meilleurs résultats que lorsque la silicone est introduite dans la phase aqueuse de dispersion des vésicules après formation de celles-ci.

La présente invention a donc pour premier objet un procédé de fabrication d'une composition cosmétique pour application sur les cheveux contenant au moins un lipide, au moins une silicone et de l'eau, caractérisé par le fait que l'on mélange avec de l'eau au moins un lipide amphiphile ionique susceptible de former des vésicules avec au moins une silicone, que l'on soumet ce mélange à un procédé de formation de vésicules encapsulant une phase aqueuse, et que l'on effectue une dispersion du produit obtenu dans une phase aqueuse de dispersion.

De préférence, la phase aqueuse de dispersion contient au moins agent tensioactif cationique.

Le procédé de formation de vésicules utilisé est, de préférence, un procédé ne nécessitant pas de solvant, dans lequel on effectue une fusion du mélange de lipide(s) amphiphile(s) ionique(s) et de silicone(s), on introduit une phase aqueuse à encapsuler de façon à former une phase lamellaire hydratée, on poursuit l'addition de cette phase sous agitation énergique pour former des vésicules, puis on ajoute une phase aqueuse de dispersion.

On peut également utiliser un procédé dans lequel on dissout le(s) lipide(s) amphiphile(s) ionique(s) et la(les) silicone(s) dans un solvant organique; dans le récipient où est placée la solution ainsi obtenue, on évapore le solvant pour former, sur les parois dudit récipient, un film du mélange (lipide(s)/silicone(s)); on ajoute dans ledit récipient la phase aqueuse à encapsuler, sous agitation énergique, jusqu'à formation des vésicules; on ajoute enfin une phase aqueuse de dispersion.

Le lipide amphiphile ionique utilisé selon la présente invention est avantageusement choisi parmi les composés suivants :
a) les phospholipides naturels ou synthétiques, notamment la lécithine d'oeuf ou de soja, la sphingemyéline, la dipalmitoyl-phosphatidylcholine ou la lécithine hydrogénée ;
b) les composés amphotères comportant deux chaînes lipophiles ou une association de deux ions organiques à longue chaîne de signes opposés ;
c) les composés anioniques.

Parmi les composés anioniques, on choisit avantageusement ceux de formule : 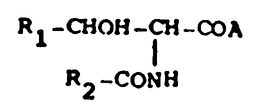 formule dans laquelle :
- R₁ désigne un radical alcoyle ou alcényle en C₇ -C₂₁;
- R₂ désigne un radical hydrocarboné, saturé ou insaturé, en C₇ -C₃₁;
- COA désigne un groupement choisi parmi les groupements suivants :
   . COOM, M étant H, Na, K, NH₄ ou un ion ammonium substitué dérivé d'une amine ;
   . un reste 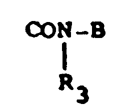 B étant un radical dérivé d'amines primaires ou secondaires, mono- ou polyhydroxylées et R₃ désignant un atome d'hydrogène ou un radical méthyle, éthyle ou hydroxyéthyle ;
   . un reste 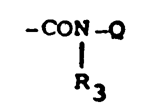 Q désignant un radical amino-alcoyle ou ammonioalcoyle substitué et R₃ ayant la signification indiquée ci-dessus ; et
   . COOZ, Z représentant le reste d'un polyol en C₃ -C₇.

De façon connue, ces lipides ioniques peuvent être associés à au moins un additif stabilisant destiné à modifier la perméabilité ou la charge superficielle des feuillets lipidiques de la phase lamellaire lipidique hydratée. Selon l'invention, ces additifs sont plus particulièrement choisis dans le groupe formé par les stérols, tels que le cholestérol ou le bêta-sitostérol ; les sels monosodiques ou disodiques de glutamates d'acyle, le radical acyle étant en C₁₄-C₂₂, tel que le sel monosodique du glutamate de stéaroyle, les sels disodiques de glutamates de cocoyle, de stéaroyle ou de mélanges de radicaux acylés dérivés du coprah et du suif, des esters phosphoriques d'alcools gras en C₁₂-C₁₆ et des tensioactifs lipophiles, tels que des phytostérols oxyéthylénés.

Les stabilisants anioniques sont associés aux composés lipidiques amphiphiles ioniques en une quantité ne dépassant, de préférence, pas 12 % en poids par rapport au poids du(ou des) lipide(s) amphiphile(s) ionique(s) constituant la phase lamellaire lipidique hydratée. Cependant pour les stérols, et notamment le cholestérol, cette même proportion peut aller jusqu'à être égale à 100 % en poids.

La(les) silicone(s) mélangée(s) aux lipides amphiphiles ioniques, selon la présente invention, est(sont), avantageusement, choisie(s) parmi les :
- polydiméthylsiloxanes et leurs mélanges avec un triméthylsiloxysilicate ;
- polydiméthylsiloxanes modifiés par des groupes hydroxyle en bout de chaîne ;
- polydiméthylsiloxanes modifiés par des groupements alcoxy en C₁₂-C₂₂ ;
- polydiméthylsiloxanes modifiés par des groupements polyoxyalkylénés, le radical alkylène étant en C₂ ou C₃ ;
- polydiméthylsiloxanes modifiés par des radicaux acyloxyalkyle dans lesquels le radical acyle est en C₁₂ - C₂₂ et le radical alkyle en C₁- C₄ ;
- polyméthylphénylsiloxanes, polyméthylalkyl (C₁- C₂₀)siloxanes ;
- polyméthyl [alkyl(C₁- C₄ )aryl]siloxanes modifiés par des groupements alkyl(C₁- C₄)amine ; et
- cyclopolysiloxanes.

Parmi les silicones avantageusement utilisées, on peut citer celles vendues sous les dénominations commerciales suivantes :
- "SILBIONE 47V500000" par la société "RHONE POULENC" (polydiméthylsiloxane ayant un poids moléculaire d'environ 250000) ;
- "FLUID DOW CORNING 593" par la société "DOW CORNING" ou "SS 4267 SILICONE FLUID" vendu par la société "GENERAL ELECTRIC CORP." (mélange de polydiméthylsiloxane et de triméthylsiloxysilicate) ;
- "SILICONE COPOLYMER F 555" par la société "S.W.S. SILICONES CORP." (stéaroxypolydiméthylsiloxane) ;
- "RHODORSIL HUILE 70633V30" par la société "RHONE POULENC" (polyméthylphénylsiloxane) ;
- "GP 7100 SILICONE FLUID" par la société "GENESEE POLYMERS CORP." (polyméthyl(alkylaryl)siloxane modifié par des groupements alkylamine) ;
- "VOLATILE SILICONE FZ 3109" vendu par la société "UNION CARBIDE" (tétraméthyltétraoctylcyclotétrasiloxane).

Les silicones utilisées peuvent être sous forme d'huiles, de gommes ou de résines insolubles dans l'eau, volatiles ou non.

On utilise, de préférence, au moins une silicone non volatile telle qu'une huile de polydiméthylsiloxane hydroxylé en bout de chaîne ou une gomme de polyphénylméthylsiloxane, sous forme de solution dans au moins une huile de silicone volatile cyclique du type cyclométhicone.

On utilise, plus particulièrement, les solutions suivantes :
- 15 % en poids d'un polydiméthylsiloxane non volatil tel que celui vendu sous la dénomination commerciale "SILBIONE 47V500000" par la société "RHONE POULENC" en solution dans 85 % en poids de décaméthylcyclopentasiloxane tel que celui vendu sous la dénomination commerciale "VOLATILE SILICONE 7158" par la société "UNION CARBIDE" ;
- 13 % en poids d'un mélange de polydiméthylsiloxanes non volatils hydroxylés en bout de chaîne tel que celui vendu sous la dénomination commerciale "Q2 1401" par la société "DOW CORNING" en solution dans 87 % en poids d'un mélange de décaméthylcyclopentasiloxane et d'octaméthylcyclotétrasiloxane ;
- 15 % en poids d'une gomme de phénylméthylsiloxane non volatile ayant un poids moléculaire de 600000 environ en solution dans 85 % en poids d'un mélange de décaméthylcyclopentasiloxane et d'octaméthylcyclotétrasiloxane.

L'agent tensioactif cationique éventuellement contenu selon l'invention dans la phase aqueuse de dispersion est généralement dispersé à chaud dans l'eau. Cette phase est ensuite mélangés à la dispersion contenant principalement le lipide ionique et la silicone. Le mélange est effectué soit à chaud, soit à la température ambiante. L'agent tensioactif est avantageusement un agent tensioactif insolub'le dans l'eau à température ambiante. Il est, de préférence, au moins un dérivé d'ammonium quaternaire de formule II : 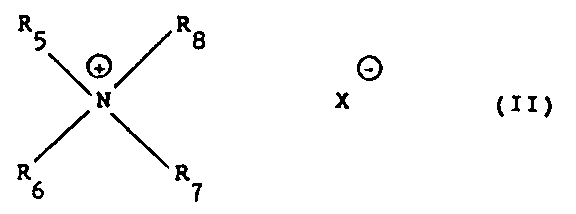 formule dans laquelle X est le chlore ou CH₃SO₄ et R₅ est un radical alkyle en C₁- C₄, de préférence le radical méthyle, et dans laquelle :
. ou bien R₆ et R₇ sont des radicaux alkyle en C₁ - C₄, identiques ou différents de R₅ et entre eux, et R₈ est un radical alkyle en C₂₀- C₂₂;
. ou bien R₆ = R₅ et R₇ = R₈ = radical alkyl en C₁₈;
. ou bien R₆ désigne un radical (alkyle et/ou alkényle) amidoéthyle dans lequel le radical alkyle et/ou alkényle est en C₁₃- C₂₁ et dérive des acides gras du suif et R₇ et R₈ forment ensemble avec l'azote un hétérocycle 4,5-dihydroimidazole substitué, notamment en position 2, par un radical alkyle et/ou alkényle en C₁₃ - C₂₁ .

L'agent tensioactif est plus particulièrement un chlorure de tétraalkylammonium de formule (II), dans lequel R₅, R₆ et R₇ sont des radicaux alkyle identiques en C₁ - C₄ , de préférence méthyle, et R₈ est un radical alkyle en C₂₀ - C₂₂ . On peut avantageusement utiliser le chlorure de béhényltriméthylammonium.

On peut également avantageusement utiliser le chlorure de distéaryldiméthylammonium, composé de formule (II) dans lequel R₅ = R₆ = CH₃ et R₇ = R₈ = alkyle en C₁₈.

Lorsque l'agent tensioactif est un méthyl-sulfate, il est avantageusement le composé de formule III : 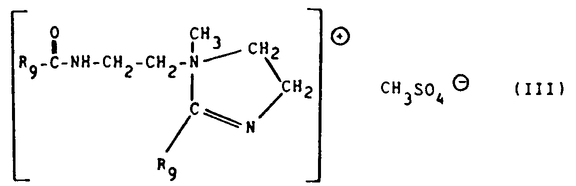 formule dans laquelle R₉ désigne un mélange de radicaux alkényle et/ou alkyle en C₁₃-C₂₁dérivé des acides gras du suif, par exemple les produits vendus sous les dénominations commerciales "REWOQUAT (W 75, W 75 PG, W 90, W 90 DPG, W 1599, W75 H)" par la société "REWO".

Selon la présente invention, on peut ajouter de façon connue au(x) lipide(s) amphiphile(s) ionique(s) et/ou à la(aux) silicone(s), avant la formation de vésicules, au moins un actif liposoluble cosmétique et/ou pharmaceutique, qui sera dans les feuillets lipidiques des vésicules. On peut également introduire, dans la phase aqueuse à encapsulér et/ou dans la phase aqueuse de dispersion, au moins un actif cosmétique et/ou pharmaceutique soluble dans l'eau. Parmi les actifs, on peut citer la vitamine A acide, l'acide linoléique, les tocophérols et les agents antichute ou de repousse des cheveux, les agents anti-pelliculaires, les retinoïdes ou apparentés, les anti-inflammatoires, les antifongiques, les anti-séborrhéiques, les filtres solaires ou analogues.

Parmi les additifs, on peut citer les conservateurs, les colorants, les parfums ou analogues. Dans la phase aqueuse de dispersion, on peut, en particulier, de façon connue, introduire un épaississant. Les agents épaississants sont plus particulièrement choisis parmi les dérives cellulosiques comme l'hydroxyméthylcellulose, la carboxyméthylcellulose, l'hydroxybutylcellulose, l'hydroxypropylcellulose et plus particulièrement l'hydroxyéthylcellulose, tels que les produits vendus sous la dénomination commerciale "NATROSOL" (150, 250) par la société "HERCULES" ou "CELLOSIZE" (QP et WP) par la société "UNION CARBIDE" ou "NATROSOL PLUS GRADE 330 CS" par la société "AQUALON", la méthylhydroxypropylcellulose, en particulier les produits vendus sous la dénomination "METHOCEL" (E, F, J, K) par la société "DOW CHEMICAL" ou les hétérobiopolysaccharides, tels que, par exemple, les gommes de xanthane commercialisées sous les marques "KELTROL" et "KELZAN" par la société "KELCO", "RHODOPOL" et "RHODIGEL" par la société "RHONE POULENC", ou "ACTIGUM" par la société "CECA/SATIA"

Ces agents épaississants peuvent être, selon l'invention, incorporés indifféremment dans des compositions contenant ou non un tensioactif cationique.

Lorsque les compositions ne contiennent pas de tensioactif cationique, on peut également employer à titre d'agent épaississant les acides polyacryliques réticulés tels que les produits vendus sous la dénomination commerciale "CARBOPOL" par la société "GOODRICH", comme les "Carbopol 910, 934, 934P, 940, 941, 1342".

Il est également possible d'introduire de façon connue, dans la phase aqueuse de dispersion une substance non-miscible à l'eau telle qu'une huile.

La présente invention a pour deuxième objet une composition obtenue par le procédé ci-dessus défini, comprenant au moins une silicone et au moins un lipide amphiphile ionique sous forme de vésicules dispersées dans une phase aqueuse de dispersion; cette composition contient également, de préférence, au moins un tensioactif cationique.

La composition selon l'invention contient, avantageusement, calculés en poids par rapport au poids total de la composition :
- 1 à 10 % d'agent(s) tensioactif(s) cationique(s) ;
- 1,5 à 20 % de lipide(s) amphiphile(s) ionique(s) ;
- 0,5 à 10 % de silicone(s) ; et, de préférence :
- 1 à 7 % d'agent(s) tensioactif(s) ;
- 1,5 à 10 % de lipide(s) amphiphile(s) ionique(s) ;
- 1,5 à 5 % de silicone(s).

La composition selon l'invention se présente sous forme de crème ou de lotion.

Les compositions selon l'invention sont utilisées, de préférence, sous forme de produits à rincer, avant et plus particulièrement après un shampooing, avant et plus particulièrement après une coloration ou une décoloration, avant et plus particulièrement après une permanente ou un défrisage. On a constaté que ces compositions sont stables au cours du temps, même en présence des agents tensioactifs cationiques, ce qui n'étant aucune prévisible par l'homme de métier, compte tenu notamment des indications contenues dans le brevet US-A- 3 957 971 (colonne 11, ligne 1). En outre, ces compositions présentent un ensemble de propriétés cosmétiques avantageuses sur l'art antérieur et d'autant plus intéressant et surprenant que leur effet est immédiat, c'est-à-dire qu'il n'est pas nécessaire pour l'utilisateur de laisser poser la composition avant de rincer, d'où un gain de temps très appréciable et une utilisation plus commode. Il demeure néanmoins possible de laisser la composition quelque temps avant de rincer et ceci sans modifier les bonnes propriétés obtenues.

Ces compositions ne graissent pas les cheveux, ne les ramollissent pas, les démêlent aisément et permettent un peignage facile à l'état humide ou sec.

Lesdites compositions confèrent, en outre, aux cheveux, des propriétés surprenantes telles qu'un lissage uniforme, une légèreté et une grande douceur de la racine à la pointe. On constate un effet surprenant d'individualisation des fibres capillaires et une diminution importante de l'électricité statique. Ces propriétés sont obtenues sur les cheveux naturels ou peu sensibilisés lorsque la imposition ne contient pas d'agent tensioactif cationique et également sur cheveux sensibilisés lorsque la composition contient un agent tensioactif cationique.

Ces compositions s'éliminent également très facilement au rinçage à l'eau.

La présente invention a pour troisième objet un procédé de traitement cosmétique de la chevelure, caractérisé par le fait que l'on applique sur celle-ci une quantité efficace de la composition selon l'invention, qu'éventuellement on geigne la chevelure et qu'enfin on rince ladite chevelure.

Selon ce traitement, on applique des quantités de composition, en général, de l'ordre de 5 à 40 g par tête.

Les exemples donnés ci-dessous, à titre illustratif et non limitatif, permettent 'de mieux comprendre l'invention.

### EXEMPLE 1

Dans une première étape, on prépare un constituant (A) comprenant les vésicules. On mélange, en agitant doucement à une température de 60°C, 5 g de lécithine de soja avec 75 % de phosphatidylcholine vendue par la société "SEPPIC" sous la dénomination "LIPOID S 75" et 3 g d'un polydiméthylsiloxane, hydroxylé en bout de chaîne (PM=600.000) (15 %) associé à un mélange (50/50) d'octaméthylcyclotétrasiloxane et de décaméthylcyclopentasiloxane (85 %), et ceci jusqu'à homogénéisation parfaite (5mn).

On introduit dans le mélange fondu 16 g d'eau portée à 80°C contenant un conservateur et l'on mélange pendant environ 5 mn ; on laisse gonfler le mélange pendant une heure. A la phase ainsi obtenue, on ajoute 24 g d'eau à 20°C ; on agite le mélange pendant quelques minutes ; puis on affine le mélange par un passage à 500 bars dans un homogénéiseur de type "Rannie". On complète par 11,6 g d'eau à 20°C sous agitation.

Dans une seconde étape, on prépare un constituant (B) comprenant la phase aqueuse de dispersion: on dissout à 80°C, 1,5 g de chlorure de distéaryl diméthylammonium dans 38,2 g d'eau pendant 10 minutes. On laisse refroidir jusqu'à 50°C.

On réalise le mélange du constituant (A) et du constituant (B) et on homogénéise sous agitation douce. Lorsque la température atteint environ 40°C, on ajoute le parfum et un conservateur puis on complète à 100 g par de l'eau à température ambiante et l'on agite doucement jusqu'à retour à la température ambiante.

On obtient une composition stable dans le temps.

On applique cette composition à raison d'environ 12 g sur des cheveux sensibilisés, lavés et essorés. On rince abondamment à l'eau. Les cheveux humide se démêlent aisément, sont lisse et doux, de la racine à la pointe. Après séchage, ils sont nerveux, ils se peignent très facilement, ils ne sont pas électriques ; ils sont brillants, lisses et déliés sur toute leur longueur. La coiffure est légère et gonflante.

### EXEMPLE 2

Dans une première étape, on prépare un constituant (A) comprenant les vésicules. On mélange, en agitant doucement à une température de 60°C, un mélange de 1,75 g de phospholipides de soja vendu par la société "NATTERMANN" sous la dénomination "PHOSPHO-LIPON 80" et 2 g d'un mélange de 15 % en poids de polyphénylméthylsiloxane (PM=600.000) dans 85 % en poids d'un mélange (50/50) d'octaméthylcyclotétrasiloxane et de décaméthylcyclopentasiloxane et ceci jusqu'à homogénéisation parfaite (5 mn).

On introduit dans le mélange fondu 7,5 g d'eau portée à 80°C contenant un conservateur et l'on mélange pendant environ 5 mn ; puis on laisse gonfler le mélange pendant 1 heures. A la phase ainsi obtenue, on ajoute 11,25 g d'eau à 20°C ; on agite le mélange pendant quelques minutes ; puis on affine le mélange par un passage à 500 bars dans un homogénéiseur de type "Rannie". On complète par 37 g d'eau à 20°C sous agitation.

Dans une seconde étape, on prépare un constituant (B) comprenant la phase aqueuse de dispersion: on dissout à 80°C, 2,8 g de chlorure de béhényl triméthyl ammonium, à 80 % de matière active dans un mélange eau/ isopropanol (15/85), dans 30 g d'eau pendant 10 minutes. On laisse refroidir jusqu'à 50°C.

On réalise le mélange du constituant A et du constituant (B) et on homogénéise sous agitation douce. Lorsque la température atteint environ 40°C, on ajoute le parfum et un conservateur, puis on complète à 100 g par de l'eau à température ambiante en agitant doucement jusqu'à retour à la température ambiante.

On obtient une composition stable, qui a les mêmes propriétés cosmétiques sur les cheveux que la composition de l'exemple 1.

### EXEMPLE 3

Dans une première étape, on prépare un constituant (A) comprenant les vésicules. On fond, en agitant doucement à une température de 85°C, 0,92 g de phytostérol polyoxyéthyléné à 5 moles d'oxyde d'éthylène vendu par la société "NIKKO" sous la dénomination "GENEROL 122 E 5". Puis, au mélange fondu, on additionne un mélange de 1,38 g de lécithine hydrogénée à 30-35 % de phosphatidylcholine hydrogénée, vendu par la société "NIKKO" sous la dénomination "LECINOL S 10" avec 0,75 g d'un polydiméthylsiloxane, vendu sous la dénomination "SILBIONE 47 V 500000" par la société "RHONE POULENC" et 4,25 g de décaméthylcyclopentasiloxane, et ceci jusqu'à homogénéisation parfaite (5 mn).

On introduit dans le mélange indu 14,6 g d'eau portée à 80°C contenant un conservateur et l'on mélange pendant environ 5 mn ; puis on laisse gonfler le mélange pendant 1 heure. A la phase ainsi obtenue, on ajoute 21,9 g d'eau à 20°C ; on agite le mélange pendant quelques minutes ; on affine le mélange par un passage à 500 bars dans un homogénéiseur de type "Gaulin" ; puis on complète par 16,45 g d'eau à 20°C sous agitation.

Dans une seconde étape, on prépare un constituant (B) comprenant la phase aqueuse de dispersion: on dissout à 80°C, 5 g (en matière active) d'un sel d'ammonium quaternaire (en solution dans le propylèneglycol à environ 75 % de matière active) vendu par la société "REWO" sous la dénomination commerciale "REWOQUAT W 75 PG" dans 18 g d'eau pendant 10 minutes. On laisse refroidir jusqu'à 50°C.

On réalise le mélange du constituant (A) et du constituant (B) et on homogénéise sous agitation douce. Lorsque la température atteint environ 40°C, on ajoute le parfum et un gel aqueux bien homogène constitué de 0,25 g d'hydroxyéthylcellulose vendue sous la dénomination "NATROSOL PLUS GRADE 330 CS" par la société "AQUALON" dissous dans 15 g d'eau contenant un conservateur ; puis on complète à 100 g par de l'eau à température ambiante et l'on agite doucement jusqu'à retour à la température ambiante.

On obtient une composition stable, qui a les mêmes propriétés cosmétiques sur les cheveux que la composition de l'exemple 1.

### EXEMPLE 4

Dans cet exemple, on a comparé l'activité de deux compositions aqueuses A et B contenant les mêmes quantités des mêmes constituants lipide, silicone et tensioactif cationique :
- une composition A, conforme à l'invention, pour laquelle la silicone a été mélangée au lipide amphiphile ionique avant la formation de vésicules ;
- une composition B, non conforme à l'invention, dans laquelle la silicone a été introduite dans la phase de dispersion.

Les compositions A et B correspondent à la formulation pondérale suivante :

| | |
|---|---|
| - Phytostérol polyoxyéthyléné à 5 moles d'oxyde d'éthylène vendu sous la dénomination "GENEROL 122 E 5" par la société "NIKKO" | 0,92 g |
| - Lécithine hydrogénée à 30-35 % de phosphatidylcholine hydrogénée vendue sous la dénomination "LECINOL S10" par la société "NIKKO" | 1,38 g |
| - Silicone vendue sous la dénomination "Q2-1401" par la société "DOW-CORNING" | 2,90 g |
| - Tensioactif cationique de formule (III) vendu sous la dénomination "REWOQUAT W75 PG" par la société "REWO" (en poids de matière active) | 2,00 g |
| - Conservateur qs | |
| - Eau qsp | 100,00 g |

On a appliqué simultanément les compositions A et B à raison de 6 g par demi-tête sur des cheveux sensibilisés, lavés et essorés. On rince abondamment l'ensemble de la chevelure, on peigne les cheveux et on les sèche.

On constate qu'après l'application, la répartition de la composition A est meilleure et, qu'une fois séchés, les cheveux traités avec la composition A selon l'invention présentent des propriétés cosmétiques supérieures quant au gonflant, à la nervosité et à la légèreté.

### EXEMPLE 5

Dans cet exemple, on a comparé l'activité de deux compositions aqueuses C et D contenant les mêmes quantités des mêmes constituants lipide et silicone :
- une composition C, conforme à l'invention, pour laquelle la silicone a été mélangée au lipide amphiphile ionique avant la formation des vésicules ;
- une composition D, non conforme à l'invention, pour laquelle la silicone a été introduite dans la phase de dispersion.

Les compositions C et D correspondent à la formulation pondérale suivante :

| | |
|---|---|
| - Phytostérol polyoxyéthyléné à 5 moles d'oxyde d'éthylène vendu sous la dénomination "GENEROL 122 E 5" par la société "NIKKO" | 3,0 g |
| - Lécithine hydrogénée à 30-35 % de phosphatidylcholine hydrogénée vendue sous la dénomination "LECINOL S10" par la société "NIKKO" | 4,5 g |
| - Silicone vendue sous la dénomination "Q2-1401" par la société "DOW CORNING" | 4,0 g |
| - Conservateur qs | |
| - Eau qsp | 100,0 g |

On a appliqué simultanément les compositions C et D à raison de 6 g par demi-tête sur des cheveux sensiblisés, lavés et essorés. On rince abondamment l'ensemble de la chevelure, on peigne les cheveux et on les sèche.

On constate qu'après l'application, les cheveux traités avec la composition C sont plus lisses et plus légers ; que le rinçage des cheveux traités avec la composition C est plus facile et qu'une fois séchés les cheveux traités avec la composition C sont plus brillants, plus gonflants et plus déliés sur toute leur longueur qu'avec la composition D.

## Revendications

1. Procédé de fabrication d'une composition cosmétique pour application sur les cheveux contenant au moins un lipide, au moins une silicone et de l'eau, caractérisé par le fait que l'on mélange avec de l'eau au moins un lipide amphiphile ionique susceptible de former des vésicules avec au moins une silicone, que l'on soumet ce mélange à un procédé de formation de vésicules encapsulant une phase aqueuse, et que l'on effectue une dispersion du produit obtenu dans une phase aqueuse de dispersion.

2. Procédé selon la revendication 1, caractérisé par le fait que la phase aqueuse de dispersion contient au moins un agent tensioactif cationique.

3. Procédé selon l'une des revendications 1 ou 2, caractérisé par le fait que l'on effectue une fusion du mélange de lipide(s) amphiphile(s) ionique(s) et de silicone(s), on intro et une phase aqueuse à encapsuler sous agitation pour former des vésicules, puis on ajoute une phase aqueuse de dispersion.

4. Procédé selon l'une des revendications 1 ou 2, caractérisé par le fait que l'on dissout le(s) lipide(s) amphiphile(s) ionique(s) et la (les silicone(s) dans un solvant organique, que, dans le récipient où est placée la solution ainsi obtenue, on évapore le solvant, que l'on ajoute dans ledit récipient la phase aqueuse à encapsuler, sous agitation, jusqu'à formation des vésicules, et que l'on ajoute enfin une phase aqueuse de dispersion.

5. Procédé selon l'une des revendications 1 ou 2, caractérisé par le fait que :
a) dans une première étape, on dissout le(s) lipide(s) amphiphile(s) ionique(s) et la (les) silicone(s) dans au moins un solvant organique non miscible à l'eau ;
b) dans une deuxième étape, on ajoute la phase organique obtenue à la première étape à une phase aqueuse en quantités telles que la dispersion obtenue a l'étape suivante est une dispersion du type huile - dans - eau ;
c) dans une troisième étape, on disperse le mélange de la deuxième étape sous agitation ;
d) dans une quatrième étape, on évapore le solvant en même temps qu'une partie de l'eau ; et que dans les étapes c) et d) la phase continue de la dispersion reste en permanence la phase aqueuse.

6. Procédé selon l'une des revendications 1 à 5, caractérisé par le fait que le lipide amphiphile ionique est choisi dans le groupe formé par :
a) les phospholipides naturels ou synthétiques, notamment la lécithine d'oeuf ou de soja, la sphingomyéline, la dipalmitoyl-phosphatidylcholine ou la lécithine hydrogénée ;
b) les composés amphotères comportant deux chaînes lipophiles ou une association de deux ions organiques à longue chaîne de signes opposés ;
c) les composes anioniques.

7. Procédé selon la revendication 6 , caractérisé par le fait que le(s) lipide(s) amphiphile(s) ionique(s) est (sont) choisi(s) dans le groupe formé par les composés de formule 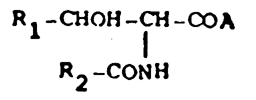 formule dans laquelle :
- R₁ désigne un radical alcoyle ou alcényle en C₇ -C₂₁;
- R₂ désigne un radical hydrocarboné, saturé ou insaturé, en C₇ -C₃₁;
- COA désigne un groupement choisi parmi les groupements suivants :
. COOM, M étant H, Na, K, NH₄ ou un ion ammonium substitué dérivé d'une amine ;
. un reste 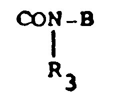 B étant un radical dérivé d'amines primaires ou secondaires, mono- ou polyhydroxylées et R₃ désignant un atome d'hydrogène ou un radical méthyle, éthyle ou hydroxyéthyle ;
. un reste 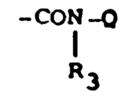 Q désignant un radical amino-alcoyle ou ammonioalcoyle substitué et R₃ ayant la signification indiquée ci-dessus ; et
. COOZ, Z représentant le reste d'un polyol en C₃ -C₇ .

8. Procédé selon l'une des revendications 1 à 7, caractérisé par le fait que le(s) lipide(s) amphiphile(s) ionique(s) est (ou sont) associé(s) Q au moins un additif stabilisant destiné à modifier la perméabilité ou la charge superficielle de la phase lamellaire lipidique hydratée.

9. Procédé selon la revendication 8, caractérisé par le fait que le(s) additif(s) stabilisant(s) est(sont) choisi(s) dans le groupe formé par les stérols; les sels monosodiques ou disodiques de glutamates d'acyle, le radical acyle étant en C₁₄ -C₂₂, des esters phosphoriques d'alcools gras en C₁₂-C₁₆ et des tensioactifs lipophiles.

10. Procédé selon l'une des revendications 8 ou 9, caractérisé par le fait que le(s) additif(s) stabilisant(s) est(sont) présent(s) en une quantité inférieure à 12 % en poids par rapport au poids du(des) lipide(s) amphiphile(s) ionique(s).

11. Procédé selon la revendication 9, dans lequel au moins un additif stabilisant est un stérol, caractérisé par le fait que le(les) stérol(s) est(sont) présent(s) en une proportion inférieure à 100 % en poids par rapport au poids du(des) lipide(s) amphiphile(s) ionique(s).

12. Procédé selon l'une des revendications 1 à 11, caractérisé par le fait que la(ou les) silicone(e) mélangée(e) aux lipides est(sont) choisie(s) dans le groupe formé par les:
- polydiméthylsiloxanes et leurs mélanges avec un triméthylsiloxysilicate ;
- polydiméthylsiloxanes modifiés par des groupes hydroxyle en bout de chaînes ;
- polydiméthylsiloxanes modifiés par des groupements alcoxy en C₁₂- C₂₂;
- polydiméthylsiloxanes modifiés par des groupements polyoxyalkylène, le radical alkylène étant en C₂ ou C₃ ;
- polydiméthylsiloxanes modifiés par des radicaux acyloxyalkyle où le groupement acyle est en C₁₂- C₂₂ et le radical alkyle en C₁- C₄ ;
- polyméthylphénylsiloxanes, polyméthylalkyl (C₁ - C₂₀)siloxanes ;
- polyméthyl[alkyl(C₁ - C₄)aryl]siloxanes modifiés par des groupements alkyl (C₁-C₄)amine ; et
- cyclopolysiloxanes.

13. Procédé selon l'une des revendications 1 à 12, caractérisé par le fait que l'on utilise au moins une silicone non volatile en solution dans au moins une huile de silicone volatile cyclique.

14. Procédé selon la revendication 2, caractérisé par le fait que l'agent tensioactif est au moins un dérivé d'ammonium quaternaire de formule II : 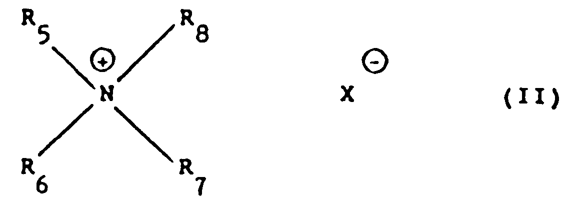 formule dans laquelle X est le chlore ou CH₃SO₄ et R₅ est un radical alkyl en C₁- C₄ et dans laquelle :
. ou bien R₆ et R₇ sont des radicaux alkyle en C₁- C₄, identiques ou différents de R₅ et entre eux, et R₈ est un radical alkyle en C₂₀- C₂₂;
. ou bien R₆ = R₅ et R₇ = R₈ = radical alkyl en C₁₈;
. ou R₆ désigne un radical (alkyl et/ou alkényl)amidoéthyle, dans lequel le radical alkyl et/ou alkényle est en C₁₃- C₂₁ et dérive des acides gras du suif et R₇ et R₈ forment ensemble avec l'azote un hétérocycle 4,5-dihydroimidazole substitué par un radical alkyle et/ou alkényle en C₁₃-C₂₁.

15. Procédé selon l'une des revendications 1 à 14, caractérisé par le fait que l'on ajoute au(s) lipide(s) amphiphile(s) ionique(s) et/ou à la (aux) silicone(s) au moine un actif liposoluble pharmaceutique et/ou cosmétique.

16. Procédé selon l'une des revendications 1 à 14, caractérisé par le fait que l'on introduit dans la phase aqueuse à encapsuler et/ou dans la phase aqueuse de dispersion au moins un actif cosmétique et/ou pharmaceutique soluble dans l'eau.

17. Procédé selon l'une des revendications 1 à 14, caractérisé par le fait que l'on introduit dans la phase aqueuse de dispersion une substance non miscible à l'eau.

18. Composition obtenue par le procédé selon l'une des revendications 1 à 17, comprenant au moins une silicone et au moins un lipide amphiphile iomique sous forme de vésicules dispersées dans une phase aqueuse de dispersion.

19. Composition selon la revendication 18, caractérisée par le fait que la phase aqueuse de dispersion contient au moins un agent tensioactif cationique.

20. Composition selon la revendication 19, caractérisée par le fait qu'elle contient, calculée en poids par rapport au poids total de la composition :
- 1 à 10 % d'agent(s) tensioactif(s) cationique(s);
- 1,5 à 20 % de lipide(s) amphiphile(s) ionique(s);
- 0,5 à 10 % de silicone(s).

21. Composition selon la revendication 20, caractérisée par le fait qu'elle contient, calculés en poids par rapport au poids total de la composition :
- 1 à 7 % d'agent(s) tensioactif(s);
- 1,5 à 10 % de lipide(s) amphiphile(s) ionique(s);
- 1,5 à 5 % de silicone(s).

22. Procédé de traitement cosmétique de la chevelure, caractérisé par le fait que l'on applique sur celle-ci une quantité efficace de la composition selon l'une des revendications 18 à 21, qu'éventuellement on peigne la chevelure et qu'enfin on rince ladite chevelure.

## Claims

1. Process for manufacturing a cosmetics composition for application to hair, which contains at least one lipid, at least one silicone and water, characterised in that at least one ionic amphiphilic lipid capable of forming vesicles with at least one silicone is mixed with water, in that this mixture is subjected to a process for forming vesicles encapsulating an aqueous phase and in that a dispersion of the product obtained is carried out in an aqueous dispersion phase.

2. Process according to Claim 1, characterised in that the aqueous dispersion phase contains at least one cationic surface-active agent.

3. Process according to one of Claims 1 or 2, characterised in that the mixture of ionic amphiphilic lipid(s) and silicone(s) is melted, an aqueous phase to be encapsulated is introduced with stirring in order to form vesicles and then an aqueous dispersion phase is added.

4. Process according to one of Claims 1 or 2, characterised in that the ionic amphiphilic lipid(s) and the silicone(s) are dissolved in an organic solvent, in that; in the container where the solution thus obtained is placed, the solvent is evaporated, in that the aqueous phase to be encapsulated is added, with stirring, into the said container until the vesicles have formed and in that, finally, an aqueous dispersion phase is added.

5. Process according to one of Claims 1 or 2, characterised in that :
a) in a first stage, the ionic amphiphilic lipid(s) and the silicone(s) are dissolved in at least one water-immiscible organic solvent;
b) in a second stage, the organic phase obtained in the first stage is added to an aqueous phase in quantities such that the dispersion obtained in the following stage is a dispersion of the oil-in-water type;
c) in a third stage, the mixture of the second stage is dispersed with stirring;
d) in a fourth stage, the solvent is evaporated at the same time as part of the water; and in that, in stages c) and d), the continuous phase of the dispersion is always the aqueous phase.

6. Process according to one of Claims 1 to 5, characterised in that the ionic amphiphilic lipid is chosen from the group formed by:
a) natural or synthetic phospholipids, particularly egg or soya lecithin, sphingomyelin, dipalmitoyl phosphatidylcholine or hydrogenated lecithin;
b) amphoteric compounds containing two lipophilic chains or a combination of two long-chain organic ions with opposite signs;
c) anionic compounds.

7. Process according to Claim 6, characterised in that the ionic amphiphilic lipid(s) is (are) chosen from the group formed by the compounds of formula 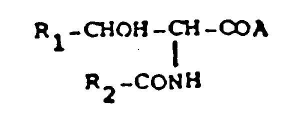 in which formula:
- R₁ denotes a C₇-C₂₁ alkenyl or alkyl radical;
- R₂ denotes a C₇-C₃₁, saturated or unsaturated hydrocarbon radical;
- COA denotes a group chosen from the following groups:
• COOM, M being H, Na, K, NH₄ or a substituted ammonium ion derived from an amine;
• a residue 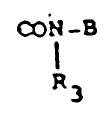 B being a radical derived from mono- or polyhydroxylated primary or secondary amines and R₃ denoting a hydrogen atom or a methyl, ethyl or hydroxyethyl radical;
• a residue 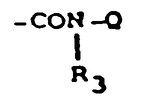 Q denoting a substituted aminoalkyl or ammonioalkyl radical and R₃ having the meaning indicated above; and
• COOZ, Z representing the residue of a C₃-C₇ polyol.

8. Process according to one of Claims 1 to 7, characterised in that the ionic amphiphilic lipid(s) is (or are) combined with at least one stabilizing additive intended to alter the permeability or the surface charge of the hydrated lipid lamellar phase.

9. Process according to Claim 8, characterised in that the stabilizing additive(s) is (are) chosen from the group formed by sterols; monosodium or disodium salts of acylglutamates, the acyl radical being C₁₄-C₂₂, phosphoric esters of C₁₂-C₁₆ fatty alcohols and lipophilic surfactants.

10. Process according to one of Claims 8 or 9, characterised in that the stabilizing additive(s) is (are) present in a quantity which is less than 12% by weight in relation to the weight of the ionic amphiphilic lipid(s).

11. Process according to Claim 9, in which at least one stabilizing additive is a sterol, characterised in that the sterol(s) is (are) present in a proportion which is less than 100% by weight in relation to the weight of the ionic amphiphilic lipid(s).

12. Process according to one of Claims 1 to 11, characterised in that the silicone(s) mixed with the lipids is (are) chosen from the group formed by the:
- polydimethylsiloxanes and their mixtures with a trimethylsiloxysilicate;
- polydimethylsiloxanes modified with hydroxyl groups at the chain ends;
- polydimethylsiloxanes modified with C₁₂-C₂₂ alkoxy groups;
- polydimethylsiloxanes modified with polyoxyalkylenated groups, the alkylene radical being C₂ or C₃;
- polydimethylsiloxanes modified with acyloxyalkyl radicals where the acyl group is C₁₂-C₂₂ and the alkyl radical is C₁-C₄;
- polymethylphenylsiloxanes, polymethyl(C₁-C₂₀)alkylsiloxanes;
- polymethyl[(C₁-C₄)alkylaryl]siloxanes modified with (C₁-C₄)alkylamine groups; and
- cyclopolysiloxanes.

13. Process according to one of Claims 1 to 12, characterised in that at least one nonvolatile silicone in solution in at least one cyclic volatile silicone oil is used.

14. Process according to Claim 2, characterised in that the surface-active agent is at least one quaternary ammonium derivative of formula II: 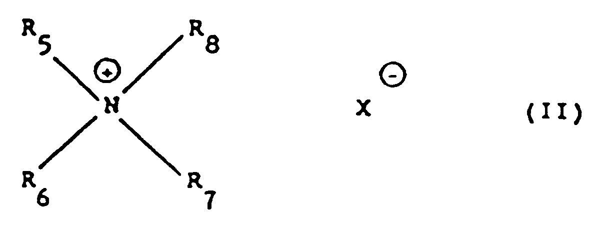 in which formula X is chlorine or CH₃SO₄ and R₅ is a C₁-C₄ alkyl radical and in which:
• either R₆ and R₇ are C₁-C₄ alkyl radicals, which are identical to or different from R₅ and each other, and R₈ is a C₂₀-C₂₂ alkyl radical;
• or R₆ = R₅ and R₇ = R₉ = C₁₈ alkyl radical;
• or R₅ denotes an (alkyl and/or alkenyl)aminoethyl radical in which the alkyl and/or alkenyl radical is C₁₃-C₂₁ and derives from the fatty acids of tallow and R₇ and R₈ together with the nitrogen form a 4,5-dihydroimidazole heterocycle which is substituted with a C₁₃-C₂₁ alkyl and/or alkenyl radical.

15. Process according to one of Claims 1 to 14, characterised in that at least one liposoluble pharmaceutical and/or cosmetic active principle is added to the ionic amphiphilic lipid(s) and/or to the silicone(s).

16. Process according to one of Claims 1 to 14, characterised in that at least one water-soluble cosmetic and/or pharmaceutical active principle is introduced into the aqueous phase to be encapsulated and/or into the aqueous dispersion phase.

17. Process according to one of Claims 1 to 14, characterised in that a water-immiscible substance is introduced into the aqueous dispersion phase.

18. Composition obtained by the process according to one of Claims 1 to 17, comprising at least one silicone and at least one ionic amphiphilic lipid in the form of vesicles dispersed in an aqueous dispersion phase.

19. Composition according to Claim 18, characterised in that the aqueous dispersion phase contains at least one cationic surface-active agent.

20. Composition according to Claim 19, characterised in that it contains, calculated by weight in relation to the total weight of the composition:
- 1 to 10% of cationic surface-active agent(s);
- 1.5 to 20% of ionic amphiphilic lipid(s);
- 0.5 to 10% of silicone(s).

21. Composition according to Claim 20, characterised in that it contains, calculated by weight in relation to the total weight of the composition:
- 1 to 7% of surface-active agent(s);
- 1.5 to 10% of ionic amphiphilic lipid(s);
- 1.5 to 5% of silicone(s).

22. Process for cosmetic treatment of the hair, characterised in that an effective quantity of the composition according to one of Claims 18 to 21 is applied to the latter, in that optionally the hair is combed and in that finally the said hair is rinsed.

## Patentansprüche

1. Verfahren zur Herstellung einer kosmetischen Zusammensetzung zur Anwendung auf den Haaren, die mindestens ein Lipid, mindestens ein Silikon und Wasser enthält, dadurch gekennzeichnet, daß man Wasser und mindestens ein amphiphiles ionisches Lipid, das mit mindestens einem Silikon Vesikeln zu bilden vermag, mischt, daß man diese Mischung einem Verfahren zur Herstellung von Vesikeln, die eine wässrige Phase einschließen, unterwirft und daß man eine Dispersion des erhaltenen Produktes in einer wässrigen Dispersionsphase herstellt.

2. Verfahren gemäß Anspruch 1, dadurch gekennzeichnet, daß die wässrige Phase der Dispersion mindestens ein oberflächenaktives, kationisches Agens enthält.

3. Verfahren gemäß einem der Ansprüche 1 oder 2, dadurch gekennzeichnet, daß man die Mischung aus dem (den) amphiphilen ionischen Lipid(en) und dem (der) Silikon(e) schmilzt, daß man unter Rühren eine einzukapselnde wässrige Phase zugibt, um Vesikeln zu bilden, und anschliessend eine wässrige Dispersionsphase zugibt.

4. Verfahren gemäß einem der Ansprüche 1 oder 2, dadurch gekennzeichnet, daß man das (die) amphiphile(n) ionische(n) Lipid(e) und das (die) Silikon(e) in einem organischen Lösungsmittel löst, daß man aus dem Gefäß, in welches die so erhaltene Lösung gegeben wird, das Lösungsmittel abdampft, daß man die einzuschließende wässrige Phase unter Rühren bis zur Vesikelbildung in das Gefäß gibt, und daß man schließlich eine wässrige Dispersionsphase zugibt.

5. Verfahren gemäß einem der Ansprüche 1 oder 2, dadurch gekennzeichnet, daß man:
a) in einer ersten Stufe das (die) amphiphile(n) ionische(n) Lipid(e) und das (die) Silikon(e) in mindestens einem nicht-wassermischbaren organischen Lösungsmittel löst;
b) in einer zweiten Stufe die in der ersten Stufe erhaltene organische Phase zu einer solchen Menge einer wässrigen Phase gibt, daß die in der folgenden Stufe erhaltene Dispersion eine Dispersion vom Typ Öl-in-Wasser ist;
c) in einer dritten Stufe die Mischung der zweiten Stufe unter Rühren dispergiert;
d) in einer vierten Stufe das Lösungsmittel gleichzeitig mit einem Teil des Wassers abdampft;
und daß in den Stufen c) und d) die kontinuierliche Phase der Dispersion auf Dauer die wässrige Phase bleibt.

6. Verfahren gemäß einem der Ansprüche 1 bis 5, dadurch gekennzeichnet, daß das amphiphile ionische Lipid aus der Gruppe der folgenden Verbindungen gewählt wird:
a) natürlichen oder synthetischen Phospholipiden, insbesondere Lecithin aus Ei oder aus Soja, Sphingomyelin, Dipalmitoylphosphatidylcholin oder hydriertem Lecithin;
b) amphoteren Verbindungen, die zwei lipophile Ketten oder eine Vereinigung von zwei entgegengesetzt geladenen langkettigen organischen Ionen aufweisen;
c) anionischen Verbindungen.

7. Verfahren gemäß Anspruch 6, dadurch gekennzeichnet, daß das (die) amphiphile(n) ionischen(n) Lipid(e) aus der Gruppe der Verbindungen der folgenden Formel gewählt wird (werden): 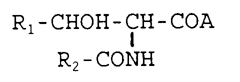 worin
- R₁ für einen C₇-C₂₁-Alkyl- oder -Alkenylrest steht;
- R₂ für einen gesättigten oder ungesättigten C₇-C₃₁-Kohlenwasserstoffrest steht;
- COA für eine Gruppe steht, die aus den folgenden Gruppen ausgewählt wird:
• COOM, worin
M = H, Na, K, NH₄ oder ein von einem Amin abgeleitetes substituiertes Ammoniumion ist;
• einen Rest 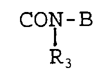 worin B ein von einem primären oder sekundären mono- oder polyhydroxylierten Amin abgeleiteter Rest ist, und
R₃ für ein Wasserstoffatom oder einen Methyl-, Ethyl- oder Hydroxyethylrest steht;
• einen Rest der Formel 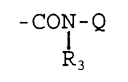 worin
Q für einen substituierten Aminoalkyl- oder Ammoniumalkyl-Rest steht, und
R₃ die oben angegebene Bedeutung hat; und
• COOZ,
worin
Z für den Rest eines C₃-C₇-Polyols steht.

8. Verfahren gemäß einem der Ansprüche 1 bis 7, dadurch gekennzeichnet, daß das (die) amphiphile(n) ionische(n) Lipid(e) mit mindestens einem stabilisierenden Additiv verbunden ist (sind), welche(s) die Permeabilität oder die Oberflächenladung der lamellaren hydratisierten Lipid-Phase modifizieren soll (sollen).

9. Verfahren gemäß Anspruch 8, dadurch gekennzeichnet, daß der (die) Stabilisator-Zusatz (-Zusätze) aus der Gruppe der folgenden Verbindungen gewählt ist (sind): Sterolen, Mono- oder Di-Natriumsalzen von Acylglutamaten mit einem C₁₄-C₂₂-Acylrest, den Phosphorsäureestern von C₁₂-C₁₆-Fettalkoholen und oberflächenaktiven Lipophilen.

10. Verfahren gemäß einem der Ansprüche 8 oder 9, dadurch gekennzeichnet, daß der (die) Stabilisator-Zusatz (-Zusätze) in einer Menge von weniger als 12 Gew.-%, bezogen auf das Gewicht des (der) der amphiphilen ionischen Lipids (Lipide) vorliegt (vorliegen).

11. Verfahren gemäß Anspruch 9, wobei mindestens einer der Stabilisatorzusätze ein Sterol ist, dadurch gekennzeichnet, daß das (die) Sterol(e) in einem Anteil von weniger als 100 Gew.-%, bezogen auf das Gewicht des (der) amphiphilen ionischen Lipids (Lipide) vorliegt (vorliegen).

12. Verfahren gemäß einem der Ansprüche 1 bis 11, dadurch gekennzeichnet, daß das (die) mit den Lipiden gemischte(n) Silikon(e) aus der Gruppe der folgenden Verbindungen gewählt ist (sind):
- Polydimethylsiloxane und ihre Mischungen mit einem Trimethylsiloxysilicat;
- Polydimethylsiloxane, modifiziert durch Hydroxylgruppen an den Kettenenden;
- Polydimethylsiloxane, modifiziert mit C₁₂-C₂₂-Alkoxygruppen;
- Polydimethylsiloxane, modifiziert mit Polyoxyalkylengruppen, mit C₂- oder C₃-Alkylenresten;
- Polydimethylsiloxane, modifiziert mit Acyloxyalkylresten, mit einer C₁₂-C₂₂-Acylgruppe und einem C₁-C₄-Alkylrest;
- Polymethylphenylsiloxane, Polymethyl(C₁-C₂₀)alkylsiloxane;
- Polymethyl[C₁-C₄-alkylaryl]siloxane, modifiziert mit C₁-C₄-Alkylamingruppen; und
- Cyclopolysiloxane.

13. Verfahren gemäß einem der Ansprüche 1 bis 12, dadurch gekennzeichnet, daß man mindestens ein nicht-flüchtiges Silikon, gelöst in mindestens einem flüchtigen, cyclischen Silikonöl, verwendet.

14. Verfahren gemäß Anspruch 2, dadurch gekennzeichnet, daß das oberflächenaktive Agens mindestens ein quaternäres Ammoniumderivat der Formel II ist: 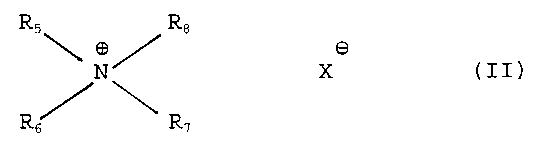 worin
X für Chlor oder CH₃SO₄ steht und R₅ für einen C₁-C₄-Alkylrest steht, und wobei:
• entweder R₆ und R₇ für C₁-C₄-Alkylreste stehen, die zu R₅ und untereinander gleich oder verschieden sind, und R₈ einen C₂₀-C₂₂-Alkylrest bedeutet;
• oder R₆ = R₅ und R₇ = R₈ = ein C₁₈-Alkylrest ist;
• oder R₆ einen (Alkyl und/oder Alkenyl)amidoethylrest darstellt, wobei es sich um einen C₁₃-C₂₁-Alkyl- und/oder -Alkenylrest, abgeleitet aus Talgfettsäuren handelt, und
R₇ und R₈ gemeinsam mit Stickstoff einen C₁₃-C₂₁-Alkyl- und/oder -Alkenylrest-substituierten 4,5-Dihydroimidazol-Heterozyklus bilden.

15. Verfahren gemäß einem der Ansprüche 1 bis 14, dadurch gekennzeichnet, daß man zu dem (den) amphiphilen ionischen Lipid(en) und/oder zu dem (den) Silikon(en) mindestens einen fettlöslichen pharmazeutischen und/oder kosmetischen Wirkstoff einbringt.

16. Verfahren gemäß einem der Ansprüche 1 bis 14, dadurch gekennzeichnet, daß man in die einzuschließende wässrige Phase und/oder in die wässrige Dispersionsphase mindestens einen wasserlöslichen kosmetischen und/oder pharmazeutischen Wirkstoff einbringt.

17. Verfahren gemäß einem der Ansprüche 1 bis 14, dadurch gekennzeichnet, daß man in die wässrige Disperisonsphase eine mit Wasser nicht mischbare Substanz einbringt.

18. Zusammensetzung, erhältlich nach einem Verfahren gemäß einem der Ansprüche 1 bis 17, die mindestens ein Silikon und mindestens ein amphiphiles, ionisches Lipid in Form von dispergierten Vesikeln in einer wässrigen Dispersionsphase enthält.

19. Zusammensetzung gemäß Anspruch 18, dadurch gekennzeichnet, daß die wässrige Dispersionsphase mindestens ein kationisches oberflächenaktives Mittel enthält.

20. Zusammensetzung gemäß Anspruch 19, dadurch gekennzeichnet, daß sie, bezogen auf das Gesamtgewicht der Zusammensetzung, enthält:
- 1 bis 10 Gew.-% kationische(s) oberflächenaktive(s) Mittel;
- 1,5 bis 20 Gew.-% amphiphile(s) ionische(s) Lipid(e);
- 0,5 bis 10 Gew.-% Silikon(e).

21. Zusammensetzung gemäß Anspruch 20, dadurch gekennzeichnet, daß sie, bezogen auf das Gesamtgewicht der Zusammensetzung, enthält:
- 1 bis 7 Gew.-% oberflächenaktive(s) Mittel;
- 1,5 bis 10 Gew.-% amphiphile(s) ionische(s) Lipid(e);
- 1,5 bis 5 Gew.-% Silikon(e).

22. Verfahren zur kosmetischen Behandlung der Haare, dadurch gekennzeichnet, daß man auf diese eine wirksame Menge einer Zusammensetzung gemäß einer der Ansprüche 18 bis 21 aufträgt, gegebenenfalls die Haare kämmt und schließlich die Haare spült.
